# EUROPEAN PATENT APPLICATION

(11) **EP 4 461 765 A1**
(43) Date of publication of application: **13.11.2024**
(21) Application number: 23803051.4
(22) Date of filing: 12.05.2023
(51) Int. Cl.: C08J 3/075, C08L 101/14, A61L 27/52

(54) **HYDROGEL, AND PREPARATION METHOD AND APPLICATION THEREFOR**

(30) Priority: 13.05.2022 IT 202200009974
(71) Applicant: Yan, Yunsong, Hengyang, Hunan 421002 (CN)
(72) Inventor: Yan, Yunsong, Hengyang, Hunan 421002 (CN)
(74) Representative: Cammareri, Emanuele
(86) International application number: PCT/CN2023/094086
(87) International publication number: WO 2023/217283

(57) **Abstract**

Provided in the present invention is a hydrogel. The hydrogel comprises water, glycerol, cellulose ether, and a polymer. The polymer comprises a first polymer and a second polymer. The first polymer is polyacrylonitrile. The second polymer is a cross-linked polyacrylic acid or a cross-linked poly N-hydroxyethyl acrylamide. Further provided are a premix for preparing the hydrogel and a method for preparing the hydrogel. Further provided is a pharmaceutical composition. The pharmaceutical composition can be at least one of a transdermal patch, a wound care patch, a skin care patch and a tissue regeneration patch, and is applied to the fields of medical biology, medical beauty, skin care, etc. Further provided is the application of the hydrogel in preparation of a sensor, an actuator, a transistor, a capacitor, a garment material and a battery. The present hydrogel has better bonding performance, mechanical performance, durability, transparency and air permeability, and also has good drug loading performance and drug sustained-release performance.

## Description

### TECHNICAL FIELD

The present invention relates to the field of material chemistry, in particular to a hydrogel, and a preparation method and application therefor.

### BACKGROUND

Hydrogels are cross-linked polymers dispersed in water and insoluble in water. The hydrogels may be prepared from natural or synthetic polymers. When dispersed in water, the hydrogel is capable of forming a three-dimensional network of polymer chains to support viscoelasticity. The hydrogel also expands with the increase of water content.

Hydrogels have been used in various biomedical applications, such as engineered scaffolds, medical diagnostic tools, therapeutic tools, drug release systems and other applications.

In particular, due to the interconnection structure inside the hydrogels, their applications in gradual release systems of pharmaceutical reagents have become possible. Therefore, through an appropriate synthesis method, a drug can be loaded into a hydrogel, and the drug will gradually migrate through the hydrogel structure until coming out.

The composition and relative quantity of monomers in a hydrogel polymer may be changed to change the diffusion and permeation characteristics of a gel containing the drug. Several methods of introducing a pharmaceutical reagent into a hydrogel have been developed. Some of them involve drug retention in the polymerization process or introduction of a hydrogel in the water swelling stage. Drug release may occur through different types of external stimuli, such as pH value change, water flowing into gel, or other mechanisms. This type of drug delivery makes hydrogels the best choice for applications such as transdermal patches and wound care patches.

However, the main disadvantage of hydrogels is their poor adhesion performance, which may be enhanced by secondary coating. In addition, another disadvantage of hydrogels is their low breaking strength, which limits the use of these materials in applications requiring special loading conditions. Another typical disadvantage of hydrogels is the lack of durability due to water loss. Moreover, the transparency and air permeability of existing hydrogels are low. Finally, it is difficult to find a compromise between the high release rate of a drug and the duration of effectiveness of a material over time.

### SUMMARY

The present invention aims to provide a hydrogel with better bonding performance, mechanical performance, durability, transparency and air permeability.

To achieve the above objective, in an aspect, a hydrogel of the present invention comprises water, glycerol, cellulose ether and a polymer; the polymer comprises a first polymer and a second polymer; the first polymer is polyacrylonitrile, and the second polymer is a cross-linked polyacrylic acid or a cross-linked poly N-hydroxyethyl acrylamide.

In another aspect, the present invention provides a premix for preparing the hydrogel. The premix comprises water, glycerol, cellulose ether, an acrylonitrile monomer, an acrylic acid monomer and a cross-linking agent monomer; or the premix includes water, glycerol, cellulose ether, an acrylonitrile monomer, an N-hydroxyethyl acrylamide monomer and a cross-linking agent monomer.

In another aspect, the present invention provides a method for preparing the hydrogel, comprising: mixing the premix with a photoinitiator, followed by photocuring.

In another aspect, the present invention provides a method for preparing the hydrogel, comprising:
- a step of preparing a first solution (3) comprising 2-hydroxyethyl cellulose (4) and water (5),
- a step of preparing a second solution (6) comprising a photoinitiator (7) for curing,
- a step of preparing a first mixture (8 or 15) comprising acrylic acid (9) or N-hydroxyethyl acrylamide (14), acrylonitrile (10), ethylene glycol dimethylacrylate (11) and glycerol (12),
- a step of mixing the first solution (3), the second solution (6), and the first mixture (8 or 15) to obtain a second mixture (13 or 16),
- a step of filling a mold (23) with the second mixture (13 or 16), the second mixture (13 or 16) is composed of a first layer (140) located in a first hydrophobic material (140a), a second layer (141) located in a second hydrophobic material (141a) on the first layer (140) and a frame (142) located on the second layer; a step of closing the mold (23) with a third layer (143) located in a third hydrophobic material (143a);
- a step of pressing the mold (23) after the closing step,
- a step of synthesizing a film (1 or 2) through photopolymerization, the photopolymerization is performed through the following step;
- exposing the second mixture (13 or 16) to ultraviolet light in the mold (23) subjected to pressure and covered by the third layer (143) to obtain the hydrogel film (1 or 2) comprising polyacrylonitrile (24),
- a step of removing the third layer (143) and the mold (23).

The present invention further provides a pharmaceutical composition, which comprises the hydrogel as described above and pharmaceutical active ingredients.

The present invention further provides application of the hydrogel as described above in preparation of a sensor, an actuator, a transistor, a capacitor, a garment material and a battery.

Through the above technical solution, the present invention provides a hydrogel with better bonding performance, mechanical performance, durability, transparency and air permeability, and good drug loading performance and drug sustained-release performance. The hydrogel can be at least one of a transdermal patch, a wound care patch, a skin care patch and a tissue regeneration patch, and is applied to the fields of medical biology, medical beauty, skin care, etc.

Other features and advantages of the present invention will be explained in detail in subsequent specific implementations.

### BRIEF DESCRIPTION OF THE DRAWINGS

The accompanying drawings are used for providing a further understanding of the present invention, constitute a part of the description, and are used together with the specific implementations below to explain the present invention, but do not constitute a limitation on the present invention. In the figures:
FIG. 1 illustrates preparation steps of a reagent mixture for film 1.
FIG. 2 illustrates preparation steps of a reagent mixture for film 2.
FIG. 3 illustrates steps of introducing a reactant mixture into a mold, polymerizing and obtaining a hydrogel film.
FIG. 4 illustrates a step of taking the hydrogel film out of the mold.
FIG. 5 illustrates an exemplary tensile photo of hydrogel film 1.

### DETAILED DESCRIPTION

Specific implementations of the present invention will be described in detail below with reference to the accompanying drawings. It should be understood that the specific implementations described here are only intended to illustrate and explain the present invention and are not intended to limit the present invention.

A first aspect of the present invention provides a hydrogel, which includes water, glycerol, cellulose ether and a polymer; the polymer includes a first polymer and a second polymer; the first polymer is polyacrylonitrile, and the second polymer is a cross-linked polyacrylic acid or a cross-linked poly N-hydroxyethyl acrylamide.

In the present invention, the cellulose ether functions as a drug coating carrier and a drug delivery channel in the hydrogel, and also as a tackifier and a water retaining agent; the polyacrylonitrile and the second polymer function to increase mechanical elasticity and drug solubility in the hydrogel. A possible reason why the above special functions are better for the bonding performance, mechanical performance, durability, transparency and air permeability of the hydrogel of the present invention lies in a mechanically stable dual network structure formed by the polyacrylonitrile and the second polymer in the hydrogel, as well as water retention and adhesion of the cellulose material, making the polymer have bonding performance, mechanical performance, durability, transparency and air permeability.

The hydrogel has a solid content of 20-35 mass%, preferably 22-25 mass%. The solid content of the hydrogel may be measured by dividing the weight of a gel solid obtained after drying by the total weight of the gel before drying.

A weight ratio of the cellulose ether to the polymer may be 1: (5-9). In order to further improve the performance of the hydrogel, the weight ratio of the cellulose ether to the polymer is preferably 1: (5-8).

In a preferred embodiment, in order to meet the requirement of better mechanical performance, the composition of the hydrogel may be adjusted to a weight ratio of 1: (7-9) between the cellulose ether and the polymer.

In another preferred embodiment, in order to meet the requirement of better viscosity, the composition of the hydrogel may be adjusted to a weight ratio of 1: (5-6) between the cellulose ether and the polymer.

The cellulose may be at least one of methyl cellulose, hydroxyethyl methyl cellulose, carboxymethyl cellulose, ethyl cellulose, benzyl cellulose, hydroxyethyl cellulose, hydroxypropyl methyl cellulose, cyanoethyl cellulose, benzyl cyanoethyl cellulose, carboxymethyl hydroxyethyl cellulose, phenyl cellulose, and nanocellulose.

In order to further improve the performance of the hydrogel, the cellulose is preferably 2-hydroxyethyl cellulose. The 2-hydroxyethyl cellulose functions to increase the viscosity of the gel and load and administrate a drug in the hydrogel. A possible reason why the above special functions are better for the bonding performance, mechanical performance, durability, transparency and air permeability of the hydrogel of the present invention lies in that its substituent group is easier to bond with water to form an internal hydrogen bond.

The 2-hydroxyethyl cellulose has a degree of molar substitution of 2-3, preferably 2.4-2.6; a 2 mass% aqueous solution of the 2-hydroxyethyl cellulose at 25°C has a viscosity 110-137 mPa•S, preferably 115-125 mPa•S.

Optionally, a weight ratio of the polyacrylonitrile to the cross-linked polyacrylic acid is 1: (5-15), preferably 1: (7-12), and more preferably 1: (9-11).

A weight ratio of the polyacrylonitrile to the cross-linked poly N-hydroxyethyl acrylamide is 1: (1-20), preferably 1: (2-10), and more preferably 1: (3-7).

Monomers of the cross-linked polyacrylic acid may include an acrylic acid monomer and a cross-linking agent monomer; and monomers of the cross-linked poly N-hydroxyethyl acrylamide may include an N-hydroxyethyl acrylamide monomer and a cross-linking agent monomer.

The cross-linking agent monomer may be at least one of ethylene glycol dimethacrylate and polyethylene glycol acrylate, preferably ethylene glycol dimethacrylate.

The acrylonitrile copolymer is a polymer obtained by photopolymerization; a photoinitiator used for the photopolymerization comprises at least one of 2,2-dimethoxy-2-phenylacetophenone, benzoin, benzoin dimethyl ether, benzoin ether, benzoin isopropyl ether, benzoin butyl ether, diphenylacetone, α,α-dimethoxy-α-phenylacetophenone, α,α-diethylacetophenone, α-hydroxyalkylbenzophenone, α-aminoalkylbenzophenone, aroyl phosphine oxide, dibenzoylphenylphosphine oxide, benzophenone, Michler's ketone and ammonium persulfate.

The concentration of the photoinitiator used for the photopolymerization may be 1-3 weight%.

The glycerol in the hydrogel may have a content of 20-40 volume%, preferably 22-31 volume%.

The present invention further provides a premix for preparing the hydrogel as described above. The premix comprises water, glycerol, cellulose ether, an acrylonitrile monomer, an acrylic acid monomer and a cross-linking agent monomer; or the premix comprises water, glycerol, cellulose ether, an acrylonitrile monomer, an N-hydroxyethyl acrylamide monomer and a cross-linking agent monomer.

The present invention further provides a method for preparing the hydrogel, comprising: mixing the premix as described above with a photoinitiator, followed by photocuring.

The present invention further provides a method for preparing the hydrogel, comprising:
- a step of preparing a first solution (3) comprising 2-hydroxyethyl cellulose (4) and water (5),
- a step of preparing a second solution (6) comprising a photoinitiator (7) for curing,
- a step of preparing a first mixture (8 or 15) comprising acrylic acid (9) or N-hydroxyethyl acrylamide (14), acrylonitrile (10), ethylene glycol dimethylacrylate (11) and glycerol (12),
- a step of mixing the first solution (3), the second solution (6), and the first mixture (8 or 15) to obtain a second mixture (13 or 16),
- a step of filling a mold (23) with the second mixture (13 or 16), the second mixture (13 or 16) is composed of a first layer (140) located in a first hydrophobic material (140a), a second layer (141) located in a second hydrophobic material (141a) on the first layer (140) and a frame (142) located on the second layer; a step of sealing the mold (23) with a third layer (143) located in a third hydrophobic material (143a);
- a step of pressing the mold (23) after the closing step,
- a step of synthesizing a film (1 or 2) through photopolymerization, the photopolymerization is performed through the following step;
- exposing the second mixture (13 or 16) to ultraviolet light in the mold (23) subjected to pressure and covered by the third layer (143) to obtain the hydrogel film (1 or 2) comprising polyacrylonitrile (24),
- a step of removing the third layer (143) and the mold (23).

The step of preparing the first solution (3) comprises dissolving 0.6 g to 1.4 g of 2-hydroxyethyl cellulose (4) in per 17 mL to 23 mL of water (5), and mechanically stirring the first solution (3) for a subsequent stage of 22 h to 26 h before mixing the phases.

The photoinitiator (7) includes 2,2-dimethoxy-2-phenylacetophenone (70).

The step of preparing the second solution (6) includes dissolving 94 mg to 106 mg of the 2,2-dimethoxy-2-phenylacetophenone (70) in per volume of 1.8 mL to 2.2 mL of ethanol (6a) within 60 min to 180 min before the mixing step.

The step of preparing the first mixture (8) includes proportionally adding the acrylic acid (9) with a volume that can vary between 475 µL and 625 µL and the acrylonitrile (10) with a volume that can vary between 60 µL and 210 µL, followed by adding (5) to the ethylene glycol dimethacrylate (11) with a volume between 18 µL and 25 µL and the glycerol (12) with a volume between 750 µL and 1200 µL; and the subsequent mixing step suitable for obtaining the second mixture (13) includes adding the first solution (3) with a volume between 1.2 mL and 3.3 mL to the first mixture (8) with a volume between 1.2 mL and 3.3 mL, followed by continuous mechanical stirring for 60 min to 180 min and addition of the photoinitiator (7) with a volume between 20 µL and 180 µL.

The filling step comprises pouring 0.8 mL to 2 mL of the second mixture (13) into the mold (23), the frame (142) has a thickness between 100 and 250 micrometers, and the synthesis stage for the photopolymerization includes ultraviolet irradiation at a wavelength between 300 nm and 380 nm for 90 min to 150 min under an intensity between 75 mw/cm² and 110 mw/cm².

The frame (142) includes polydimethylsiloxane.

The first hydrophobic material (140a) includes organic glass, the second hydrophobic material (141a) includes polytetrafluoroethylene, and the third hydrophobic material (143a) includes organic glass.

The mold (23) is also closed in the closing stage with a fourth layer (144) sandwiched between the third layer (143) and the frame (142) and including the second hydrophobic material (141a).

The present invention further provides a pharmaceutical composition, which includes the hydrogel as described above and pharmaceutical active ingredients.

The pharmaceutical composition is at least one of a transdermal patch, a wound care patch, a skin care patch and a tissue regeneration patch, and is applied to the fields of medical biology, medical beauty, skin care, etc.

The present invention further provides application of the hydrogel as described above in preparation of a sensor, an actuator, a transistor, a capacitor, a garment material and a battery.

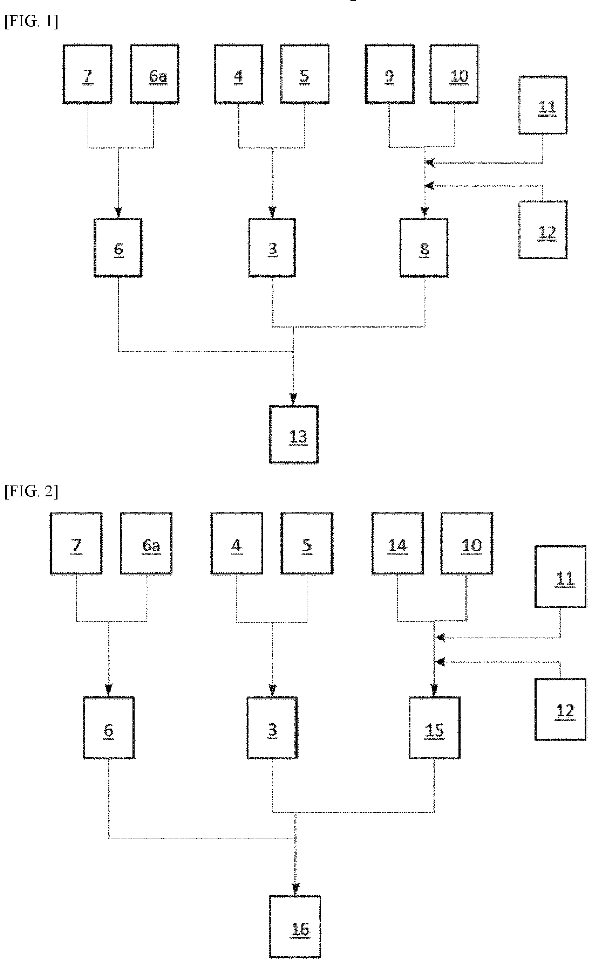

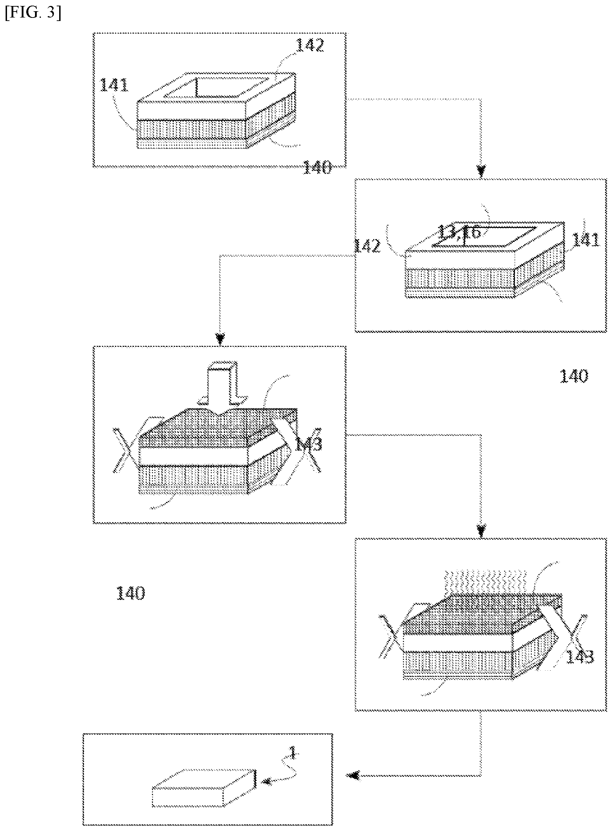

According to a particularly preferred embodiment of the present invention, a method for preparing a hydrogel film (1) comprises several steps, as shown in FIG. 1 and FIG. 3. The first step includes preparing a first solution (3) including 2-hydroxyethyl cellulose (4) and water (5). In the stage of preparing the first solution (3), it is preferred to dissolve a certain amount of 2-hydroxyethyl cellulose (4), preferably 0.6g to 1.4g, more preferably 0.8g to 1.2g, for every volume of water (5) between 17mL and 23mL, more preferably between 18mL and 21mL. Then, before the next step of the method, the first 3 solutions are mechanically mixed for 22 h to 26 h, or more preferably 23 h to 25 h. In this step, the first 3 solutions are mixed with the other prepared solutions. The 2-hydroxyethyl cellulose (4) is intended to ensure that the hydrogel has a frame structure with controlled size. The feature of the 2-hydroxyethyl cellulose (4) allows the hydrogel to be advantageously used in biomedical applications to gradually release drugs and provide sufficient adhesion capacity and water retaining capacity.

The subsequent step of the method preferably includes preparing a second solution (6) including a polymerization photoinitiator (7). The second solution (6) is required to implement the next step of polymerization, and is advantageously activated by the photoinitiator (7). The photoinitiator (7) preferably includes 2,2-dimethoxy-2-phenylacetophenone (70). The 2,2-dimethoxy-2-phenylacetophenone (70) is particularly suitable for producing lower activity substances sufficient to polymerize the monomers used in this process. The step of preparing the second solution (6) preferably includes dissolving 94 mg to 106 mg or more preferably 97 mg to 103 mg of 2,2-dimethoxy-2-phenylacetophenone (70) in per volume of ethanol 6a between 1.8 mL and 2.2mL or more preferably between 1.9 mL and 2.1 mL, for 60 min to 180 min or more preferably 90 min to 150 min before the next mixing step. The stability of the 2,2-dimethoxy-2-phenylacetophenone 70 is improved using the ethanol 6a and the time, as the substance is expected to be used for initiating the reaction.

The next step of the method includes preparing a first mixture (8) including acrylic acid (9), acrylonitrile (10), ethylene glycol dimethacrylate (11) and glycerol (12). The acrylic acid (9) and the acrylonitrile (10) are monomers that will form a final polymer structure together with the ethylene glycol dimethacrylate (11) and the glycerol (12), and induce the formation of polymer chains.

Specifically, the step of preparing the first mixture (8) preferably includes proportionally adding the acrylic acid (9) with a volume that can vary between 475 µL and 625 µL, or more preferably between 475 µL and 550 µL, or more preferably between 480 µL and 490 µL, and the acrylonitrile (10) with a volume that can vary between 60 µL and 210 µL, or more preferably between 150 µL and 190 µL, preferably followed by adding the ethylene glycol dimethacrylate (11) with a volume of 18 µL to 25 µL, or more preferably 19 µL to 24 µL, or more preferably 19 µL to 23 µL and the glycerol (12) with a volume of 750 µL to 1200 µL, or more preferably 850 µL to 1000 µL, or more preferably 900 µL to 1000 µL.

The next stage of this process includes mixing the first solution (3), the second solution (6), and the first mixture (8) to obtain a second mixture (13).

The mixing step preferably includes adding the first solution 3 with a volume between 1.2 mL and 3.3 mL, or more preferably between 1.2 mL and 2.5 mL, or more preferably between 1.6 mL and 2.5 mL to the first mixture (8), followed by mechanical stirring for preferably 60 min to 180 min, or more preferably 60 min to 90 min to 150 min. Once the mechanical mixing is completed, a certain volume of the second solution (6) is added, where the volume is preferably between 20 µL and 130 µL, or more preferably between 20 µL and 60 µL, or more preferably between 20 µL and 40 µL.

This method advantageously includes a step of filling a mold (23) with the second mixture 13, where the mold 14 is composed of a first layer 140 in a first hydrophobic material 140a, a second layer 141 on the first layer 140 and in a second hydrophobic material 141a adhered to the first layer, a second layer 142 capable of performing a synthesis platform function, and a frame 142 on the second layer 141. Therefore, the first layer 140 is a base of the mold and is preferably covered by the second layer 141, and the second closing mixture (13) is poured on the second layer 141 from the frame 142. The first layer 140 is composed of the first hydrophobic material 140a in contact with the second mixture (13) without binding.

Specifically, the first hydrophobic material 140a preferably includes organic glass, and the second hydrophobic material 141a preferably includes polytetrafluoroethylene. The two materials have the best properties to perform functions including the second mixture (13) and maintain chemical inertness requirements. The two materials can be used in thin layers. The first layer 140 and the second layer 141 preferably have two surface dimensions with a length preferably between 10 cm and 20 cm, or more preferably between 12 cm and 18 cm. The frame 142 of the mold (23) is a hollow layer in a central portion where the second mixture (13) is injected, and preferably has two horizontal dimensions same as those of the first layer 140 and the second layer 141. Specifically, the frame 142 preferably includes polydimethylsiloxane. This material provides flexibility and chemical inertness, and is easy to remove at process termination.

The filling step preferably includes pouring the second mixture (13) with a volume between 0.8 mL and 2 mL or more preferably between 1 mL and 1.7 mL into the mold (23), and the frame 142 has a thickness between 100 µm and 250 µm or more preferably between 150 µm and 230 µm. The filling stage of the mold (21) has the function of endowing the second mixture (13) with a final shape that the product must have. Once the second mixture (13) is poured, the stage for closing the mold (23) is followed by a third layer 143 made of a third hydrophobic material 143a, and the third hydrophobic material 143a is preferably organic and/or preferably solid glass in the first hydrophobic material 140a, capable of performing a covering function. The third layer 143 is appropriately placed, or preferably finely placed on the frame 142, and the frame 142 surrounds the mixture (13) therein. The covering layer has the function of endowing the final product with a correct shape in the subsequent synthesis stage, so it must ensure the same chemical inertness as other portions of the mold (23). In a variant of the closing step, the mold (23) is closed by a quarter layer 144, and the quarter layer 144 includes a hydrophobic material sandwiched between the third layer 143 and the frame 142, preferably PTFE, or more preferably composed of the second hydrophobic material 141a.

Lateral mechanical support, additional external upper or lower support, and more support can assist in mold closure. The closing step of the mold (23) is followed by a pressing step of the mold.

The mold (23) is closed and contains the mixture (13). In this stage, the mechanical pressure on the mold (23) is preferably maintained throughout the subsequent synthesis stage. In this case, the third layer (143) is appropriately placed in close contact with the mixture (13). In the procedure selection that specifies the use of the fourth layer (144), the latter is preferably in close contact with the mixture (13).

The next step includes synthesizing the film (1) through photopolymerization, where the photopolymerization is performed when the second mixture (13) is exposed to ultraviolet light in the mold (23) under pressure and covered by the third layer (143), so as to obtain the film (1) made of the hydrogel including polyacrylonitrile (24).

As expected, the polymerization occurs through reactive substances produced by the photoinitiator (7) after absorbing ultraviolet radiation. Therefore, the efficiency of the polymerization process depends on the level of ultraviolet radiation. Therefore, the reduced third thick layer (143) is preferably used to limit radiation absorption and allow the second mixture (13) to absorb most of the radiation. In the procedure that provides the addition of the fourth layer (144), the latter must also have a reduced thickness, preferably less than 200 µm. Specifically, in the synthesis stage, the wavelength of photopolymerization by irradiation with ultraviolet light is preferably between 300 nm and 380 nm or more preferably between 350 nm and 370 nm, the time is in a range of between 90 min and 150 min or more preferably between 100 min and 135 min or more preferably between 110 min and 130 min, and the intensity is in a range of between 75 mw/cm² and 110 mw/cm² or more preferably between 85 mw/cm² and 100 mw/cm².

By using these parameters, the photopolymerization process allows to obtain the film (1) with desired properties.

After the synthesis step of the hydrogel film (1), a step of removing the third layer (143) and the mold (23) is performed. During the step of removing the mold (23) for releasing the hydrogel film (1), the residue of the second mixture (13) is also removed and retained in the mold (23), and will not react in the synthesis stage. The residue must be removed as it will change the properties of the film (1).

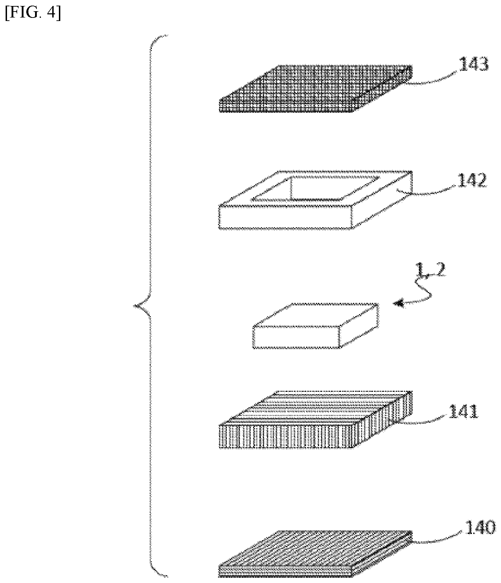

To release the film (1) produced from the mold 23, the third layer (143), the frame (142), and the second layer (141) are removed, and the first layer (140) is finally removed. FIG. 4 illustrates the stage of removing the mold (23). At the end of the process, the hydrogel film (1) is obtained through this method.

According to another particularly preferred embodiment of the present invention, the method for preparing a hydrogel film (2) includes several steps, as shown in FIG. 2 and FIG. 3. The first step includes preparing a first solution (3) including 2-hydroxyethyl cellulose (4) and water (5). In the stage of preparing the first solution (3), it is preferred to dissolve a certain amount of 2-hydroxyethyl cellulose (4), preferably 0.6g to 1.4g, more preferably 0.8g to 1.2g, for every volume of water (5) between 17mL and 23mL, more preferably between 18mL and 21mL. Then, before the next step of the method, the first 3 solutions are mechanically mixed for 22 h to 26 h, or more preferably 23 h to 25 h. In this step, the first 3 solutions are mixed with the other prepared solutions. The 2-hydroxyethyl cellulose (4) is intended to ensure that the hydrogel has a frame structure with controlled size. The feature of the 2-hydroxyethyl cellulose (4) allows the hydrogel to be advantageously used in biomedical applications to gradually release drugs and provide sufficient adhesion capacity and water retaining capacity.

The subsequent step of the method preferably includes preparing a second solution (6) including a polymerization photoinitiator (7). The second solution (6) is required to implement the next step of polymerization, and is advantageously activated by the photoinitiator (7). The photoinitiator (7) preferably includes 2,2-dimethoxy-2-phenylacetophenone (70). The 2,2-dimethoxy-2-phenylacetophenone (70) is particularly suitable for producing lower activity substances sufficient to polymerize the monomers used in this process. The step of preparing the second solution (6) preferably includes, before the next mixing step, dissolving 94 mg to 106 mg or more preferably 97 mg to 103 mg of 2,2-dimethoxy-2-phenylacetophenone (70) in per volume of ethanol 6a between 1.8 mL and 2.2 mL or more preferably 1.9 mL and 2.1 mL, for 60 min to 180 min or more preferably 90 min to 150 min. The stability of the 2,2-dimethoxy-2-phenylacetophenone (70) is improved using the ethanol 6a and the time, as the substance is expected to be used.

The next step of the method includes preparing a first mixture (15) including N-hydroxyethyl acrylamide (14), acrylonitrile (10), ethylene glycol dimethacrylate (11) and glycerol (12). The N-hydroxyethyl acrylamide (14) and the acrylonitrile (10) are monomers that will form a final polymer structure together with the ethylene glycol dimethacrylate (11) and the glycerol (12), and induce the formation of polymer chains.

Specifically, the step of preparing the first mixture (15) preferably includes proportionally adding the N-hydroxyethyl acrylamide (14) with a volume that can vary between 700 µL and 1000 µL, or more preferably between 750 µL and 950 µL, or more preferably between 720 µL and 750 µL and the acrylonitrile (10) with a volume that can vary between 60 µL and 210 µL, or more preferably between 150 µL and 190 µL, preferably followed by adding the ethylene glycol dimethacrylate (11) with a volume of 18 µL to 25 µL, or more preferably 19 µL to 24 µL, or more preferably 20 µL to 23 µL and the glycerol (12) with a volume of 750 µL to 880 µL, or more preferably 770 µL to 850 µL, or more preferably 780 µL to 1200 µL.

The next stage of this process includes mixing the first solution (3), the second solution (6), and the first mixture (15) to obtain a second mixture (16).

The mixing step preferably includes adding the first solution (3) with a volume between 1.2 mL and 2.3 mL, or more preferably between 1.2 mL and 2.5 mL, or more preferably between 1.6 mL and 2.5 mL to the first mixture (15), followed by mechanical stirring for preferably 60 min to 180 min, or more preferably 60 min to 90 min to 150 min. Once the mechanical mixing is completed, a certain volume of the second solution (6) is added, where the volume is preferably between 20 µL and 130 µL, or more preferably between 20 µL and 60 µL, or more preferably between 20 µL and 40 µL.

This method advantageously includes a step of filling a mold (23) with the second mixture (16), where the mold (23) is composed of a first layer 140 in a first hydrophobic material 140a, a second layer 141 on the first layer 140 and in a second hydrophobic material 141a adhered to the first layer, a second layer 142 capable of performing a synthesis platform function, and a frame 142 on the second layer 141. Therefore, the first layer 140 is a base of the mold and is preferably covered by the second layer 141, and the second closing mixture (16) is poured on the second layer 141 from the frame 142. The first layer 140 is composed of the first hydrophobic material 140a in contact with the second mixture (16) without binding.

Specifically, the first hydrophobic material 140a preferably includes organic glass, and the second hydrophobic material 141a preferably includes polytetrafluoroethylene. The two materials have the best properties to perform functions including the second mixture (16) and maintain chemical inertness requirements. The two materials can be used in thin layers. The first layer 140 and the second layer 141 preferably have two surface dimensions with a length preferably between 10 cm and 20 cm, or more preferably between 12 cm and 18 cm. The frame 142 of the mold 14 is a hollow layer in a central portion where the second mixture (16) is injected, and preferably has two horizontal dimensions same as those of the first layer 140 and the second layer 141. Specifically, the frame 142 preferably includes polydimethylsiloxane. This material provides flexibility and chemical inertness, and is easy to remove at process termination.

The filling step preferably includes pouring the second mixture (16) with a volume between 0.8 mL and 2 mL or more preferably between 1 mL and 1.7 mL into the mold (23), and the frame 142 has a thickness between 100 µm and 250 µm or more preferably between 150 µm and 230 µm. The filling stage of the mold 14 has the function of endowing the second mixture (16) with a final shape that the product must have. Once the second mixture (16) is poured, the stage for closing the mold (23) is followed by a third layer 143 made of a third hydrophobic material 143a, and the third hydrophobic material 143a is preferably organic and/or preferably solid glass in the first hydrophobic material 140a, capable of performing a covering function. The third layer 143 is appropriately placed, or preferably finely placed on the frame 142, and the frame 142 surrounds the mixture (16) therein. The covering layer has the function of endowing the final product with a correct shape in the subsequent synthesis stage, so it must ensure the same chemical inertness as other portions of the mold (23). In a variant of the closing step, the mold (23) is closed by a quarter layer 144, and the quarter layer 144 includes a hydrophobic material sandwiched between the third layer 143 and the frame 142, preferably PTFE, or more preferably composed of the second hydrophobic material 141a.

Lateral mechanical support, additional external upper or lower support, and more support can assist in mold closure. After the closing step of the mold (21), a pressing step of the mold (23) follows the closing step including the mixture (16). In this stage, the pressure applied by a machine to the mold (23) is preferably maintained throughout the subsequent synthesis stage. In this case, the third layer 143 is appropriately placed in close contact with the mixture (16). In a variant of the procedure that specifies the use of the fourth layer 144, the latter is preferably in close contact with the mixture (16).

The next step includes synthesizing the film (2) through photopolymerization, where the photopolymerization is performed when the second mixture (16) is exposed to ultraviolet light in the mold (23) under pressure and covered by the third layer 143, so as to obtain the film (2) made of the hydrogel including polyacrylonitrile (24).

As expected, the polymerization occurs through reactive substances produced by the photoinitiator (7) after absorbing ultraviolet radiation. Therefore, the efficiency of the polymerization process depends on the level of ultraviolet radiation. Therefore, the reduced third thick layer 143 is preferably used to limit radiation absorption and allow the second mixture 16 to absorb most of the radiation. In a variant of the procedure that provides the addition of the fourth layer 144, the latter must also have a reduced thickness, preferably less than 200 µm. Specifically, in the synthesis stage, the wavelength of photopolymerization by irradiation with ultraviolet light is preferably between 300 nm and 380 nm or more preferably between 350 nm and 370 nm, the time is in a range of between 90 min and 150 min or more preferably between 100 min and 135 min or more preferably between 110 min and 130 min, and the intensity is in a range of between 75 mw/cm² and 110 mw/cm² or more preferably between 85 mw/cm² and 100 mw/cm².

By using these parameters, the photopolymerization process allows to obtain the film (2) with desired properties.

After the synthesis step of the hydrogel film (2), a step of removing the third layer (143) and the mold (23) is performed. During the step of removing the mold (23) for releasing the hydrogel film (2), the residue of the second mixture (16) is also removed and retained in the mold (23), and will not react in the synthesis stage. The residue must be removed as it will change the properties of the film (2).

To release the film (2) produced from the mold (23), the third layer 143, the frame 142, and the second layer 141 are removed, and the first layer 140 is finally removed. FIG. 4 illustrates the stage of removing the mold (23). At the end of the process, the hydrogel film (2) is obtained through this method.

The methods for preparing the film (1) and the film (2) are simple. In addition, the film (1) and the film (2) have advantageous properties over existing hydrogel films because they are obtained by following the described procedures. The film (1) and the film (2) have better bonding performance than the existing hydrogel films. In addition to the properties produced by the interaction of carboxyl and hydroxyl functional groups, the existing hydrogel films further derive from their special secondary structural properties. The excellent performance can bring a significantly improved experience for patients with wound recovery needs (such as postoperative surgical wounds, traumatic wounds, and burn treatment) and medical applications in portions difficult to treat (such as joints and limbs).

The film (1) and the film (2) exhibit the property of gradually releasing chemical compounds, which can be better integrated into their structures than the existing films. The film (1) and the film (2) have a higher release rate, but their release efficiency does not significantly decrease over time, which is contrary to the situation in the existing films. This property is mainly due to adjustment in the pore size and gradient of the 2-hydroxyethyl cellulose (4) structure according to the adjustment method of the synthesis process. These properties make the film (1) and the film (2) particularly suitable for applications that require gradual release effects of drugs, such as transdermal patches.

In fact, in such applications, the film (1) and the film (2) allow for the examination of potential wounds in contact with them through their high optical transparency, without removing them. In addition, the film (1) and the film (2) have durability of more than 6 months under exposure to air. The film (1) and the film (2) also have good air permeability, which can create a favorable micro-environment for cell proliferation and improve the effectiveness of dressings in biomedical applications such as ointments.

Another property of the hydrogel film (1) and the film (2) is mechanical performance, which is superior to existing films in terms of breaking strength and Young's modulus. This feature makes the film (1) and the film (2) according to the present invention very advantageous, as better mechanical performance extends the application fields of the material to electronic devices, tissue engineering, adhesives, coatings, and wearable sensors. The presence of a complementary network with a polyacrylonitrile or interpenetrating system called a SNIPSy structure makes this advantage possible.

In addition, the capability of loading chemical agents further provides potential industrial applications for industry, such as a sensor, an actuator, a transistor, a capacitor, a garment material, and a battery as a support platform.

The present invention may have variations falling within the scope of invention concepts defined by the claims.

In this case, all details can be replaced with equivalent elements, and the materials, shapes, and sizes can all be arbitrary.

The present invention is further explained in detail through examples.

### Example 1

This example was used for explaining a preparation process of a hydrogel film.

A 2-hydroxyethyl cellulose aqueous solution with a concentration of 50 mg/mL was prepared. An ethanol solution of 2,2-dimethoxy-2-phenylacetophenone with a concentration of 50 mg/mL was prepared. A monomer mixture including acrylic acid, acrylonitrile and ethylene glycol dimethacrylate was prepared.

The 2-hydroxyethyl cellulose aqueous solution and the monomer mixture were mixed uniformly, the solid content was adjusted to 23 mass%, and glycerol was added to a content of 25 volume%, to obtain a pre-polymerization material, namely, a premix. A mass ratio of 2-hydroxyethyl cellulose, acrylic acid, acrylonitrile, and ethylene glycol dimethacrylate in the pre-polymerization material was 125: 508: 106: 19.

The pre-polymerization material was mixed with a photoinitiator (ethanol solution of 2,2-dimethoxy-2-phenylacetophenone, with a concentration of 2 weight%), the mixture was introduced into a mold for photopolymerization at a wavelength of 360 nm for 120 min under an intensity of 90 mw/cm², and then the mold is removed to obtain a hydrogel film 1.

### Example 2

Follow the method of Example 1, with the difference that the acrylic acid was replaced with an equal mass of N-hydroxyethyl acrylamide; and a hydrogel film 2 was obtained.

### Comparative Example 1

Follow the method of Example 1, with the difference that the acrylonitrile was replace with an equal mass of acrylic acid; and a hydrogel film C1 was obtained.

### Comparative Example 2

Follow the method of Example 1, with the difference that the 2-hydroxyethyl cellulose was replaced with an equal mass of carboxymethyl cellulose; and a hydrogel film C2 was obtained.

### Comparative Example 3

Follow the method of Example 1, with the difference that the acrylic acid was replaced with an equal mass of N-isopropylacrylamide; and a hydrogel film C3 was obtained.

### Comparative Example 4

Follow the method of Example 1, with the difference that the glycerol was replaced with an equal mass of water; and a hydrogel film C4 was obtained.

### Test Example 1

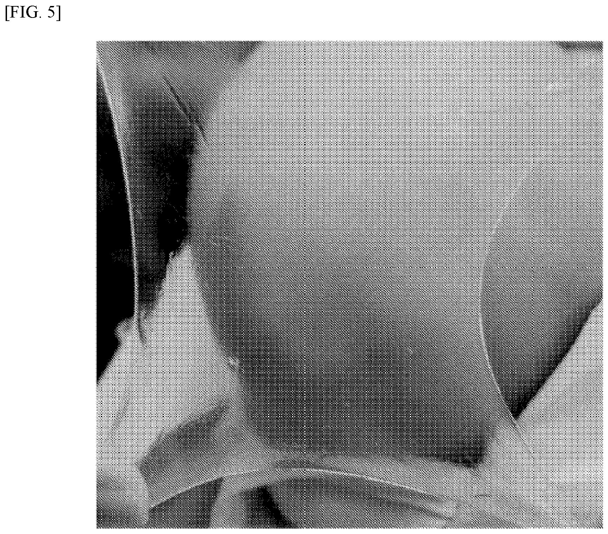

The bonding performance and mechanical performance of the hydrogel film 1 obtained in Example 1, the hydrogel film 2 obtained in Example 2, the hydrogel film C1 obtained in Comparative Example 1, and the hydrogel film C2 obtained in Comparative Example 2 were tested. The results were shown in Table 1. An exemplary tensile map of the hydrogel film 1 was as shown in FIG. 5.

**Table 1**

| Sample | Bonding performance peeling force | Mechanical performance level of stretch | Mechanical performance Young's constant |
|---|---|---|---|
| Hydrogel film 1 | 1.26-1.87 mN/mm | 620-654% | 38.54-40.27 kPa |
| Hydrogel film 2 | 2.48-3.1 mN/mm | 446-502% | 23-33 kPa |
| Hydrogel film C1 | 0.7-0.8 mN/mm | 600-694% | 90.16-92.88 kPa |
| Hydrogel film C2 | 0.72-0.75 mN/mm | 200-220% | 60-65 kPa |
| Hydrogel film C3 | Unmeasurable | Unmeasurable | Unmeasurable |
| Hydrogel film C4 | Unmeasurable | Unmeasurable | Unmeasurable |

### Test Example 2

The drug loading capacity and drug release effect of the hydrogel film 1 obtained in Example 1, the hydrogel film 2 obtained in Example 2, the hydrogel film C1 obtained in Comparative Example 1, and the hydrogel film C2 obtained in Comparative Example 2 were tested, where the drug used was lidocaine, and the results were shown in Table 2.

**Table 2**

| Sample | Drug loading capacity | Drug release effect |
|---|---|---|
| Hydrogel film 1 | >40% | Release 100% within 24 h |
| Hydrogel film 2 | >40% | Release 63% within 24 h |
| Hydrogel film C1 | >40% | Release 64% within 24 h |
| Hydrogel film C2 | >40% | Unmeasurable |
| Hydrogel film C3 | None | Unmeasurable |
| Hydrogel film C4 | None | Unmeasurable |

According to the data in Table 1 and Table 2, the present invention provides a hydrogel with better bonding performance, mechanical performance, durability, transparency and air permeability, and good drug loading performance and drug sustained-release performance.

Specifically, no gel can be formed in Comparative Example C3 or Comparative Example C4. The C2 gel was unstable and rapidly disintegrated during release, making it difficult to obtain valid data. The mixture in Comparative Example C4 hardened within less than 10 hours, indicating that the water retaining effect of the glycerol was extremely important. Moreover, the glycerol was also extremely important for the formation of a gel. In addition, from the comparison of the hydrogel film 1 with Comparative Example C3, the polyacrylic acid was crucial for the three-dimensional main structure of the gel. From the comparison of the hydrogel film 1 with Comparative Example C1, the polyacrylonitrile can significantly reduce the Young's constant of the mechanical performance of the polymer, significantly reduce the restoring force of the gel in the stretching process, and improve the mechanical performance. From the comparison of the hydrogel film 1 with Comparative Example C2, the 2-hydroxyethyl cellulose significantly improved the bonding and water retaining performance of the gel. Both the hydrogel film 1 and the hydrogel film 2 can prove that the cross-linked poly N-hydroxyethyl acrylamide and the acrylic acid were main bodies of a 3D structure of a gel.

The preferred implementations of the present invention are described in detail above in conjunction with the accompanying drawings. However, the present invention is not limited to the specific details in the above implementations. Multiple simple variations can be made to the technical solutions of the present invention within the scope of technical concepts of the present invention, and all these simple variations fall within the scope of protection of the present invention.

Furthermore, it should be noted that the various specific technical features described in the above specific implementations can be combined in any suitable way without contradiction. In order to avoid unnecessary repetition, the present invention will not further explain various possible combinations.

In addition, the various different implementations of the present invention can also be combined arbitrarily. As long as these combinations do not violate the ideas of the present invention, they should also be considered as disclosed content of the present invention.

## Claims

1. A hydrogel, wherein the hydrogel comprises water, glycerol, cellulose ether and a polymer, the polymer comprises a first polymer and a second polymer; the first polymer is polyacrylonitrile, and the second polymer is a cross-linked polyacrylic acid or a cross-linked poly N-hydroxyethyl acrylamide.

2. The hydrogel according to claim 1, wherein the hydrogel has a solid content of 20-35 mass%, preferably 22-25 mass%; and a weight ratio of the cellulose ether to the polymer is 1: (5-9), preferably 1: (5-8).

3. The hydrogel according to claim 1 or 2, wherein the cellulose ether is at least one of methyl cellulose, hydroxyethyl methyl cellulose, ethyl cellulose, benzyl cellulose, hydroxyethyl cellulose, hydroxypropyl methyl cellulose, cyanoethyl cellulose, benzyl cyanoethyl cellulose, phenyl cellulose and nanocellulose; and
the cellulose ether is preferably 2-hydroxyethyl cellulose.

4. The hydrogel according to claim 3, wherein the 2-hydroxyethyl cellulose has a degree of molar substitution of 2-3, preferably 2.4-2.6; a 5 mass% aqueous solution of the 2-hydroxyethyl cellulose at 25°C has a viscosity of 110-137 mPa·S, preferably 115-125 mPa·S.

5. The hydrogel according to claim 1, wherein a weight ratio of the polyacrylonitrile to the cross-linked polyacrylic acid is 1: (5-15), preferably 1: (7-12), and more preferably 1: (9-11); and
a weight ratio of the polyacrylonitrile to the cross-linked poly N-hydroxyethyl acrylamide is 1: (1-20), preferably 1: (2-10), and more preferably 1: (3-7).

6. The hydrogel according to claim 1, wherein monomers of the cross-linked polyacrylic acid comprise an acrylic monomer and a cross-linking agent monomer; monomers of the cross-linked poly N-hydroxyethyl acrylamide comprise an N-hydroxyethyl acrylamide monomer and a cross-linking agent monomer; and
the cross-linking agent monomer is at least one of ethylene glycol dimethacrylate and polyethylene glycol diacrylate, preferably ethylene glycol dimethacrylate.

7. The hydrogel according to claim 5, wherein the acrylonitrile copolymer is a polymer obtained by photopolymerization; a photoinitiator used for the photopolymerization comprises at least one of 2,2-dimethoxy-2-phenylacetophenone, benzoin, benzoin dimethyl ether, benzoin ether, benzoin isopropyl ether, benzoin butyl ether, diphenylacetone, α,α-dimethoxy-α-phenylacetophenone, α,α-diethylacetophenone, α-hydroxyalkylbenzophenone, α-aminoalkylbenzophenone, aroyl phosphine oxide, dibenzoylphenylphosphine oxide, benzophenone, Michler's ketone and ammonium persulfate; and preferably, the photoinitiator used for the photopolymerization is 2,2-dimethoxy-2-phenylacetophenone.

8. The hydrogel according to claim 1, wherein the glycerol in the hydrogel has a content of 20-40 volume%, preferably 22-31 volume%.

9. A premix for preparing the hydrogel according to any one of claims 1-8, wherein the premix comprises water, glycerol, cellulose ether, an acrylonitrile monomer, an acrylic acid monomer and a cross-linking agent monomer; or the premix comprises water, glycerol, cellulose ether, an acrylonitrile monomer, an N-hydroxyethyl acrylamide monomer and a cross-linking agent monomer.

10. A method for preparing the hydrogel, comprising: mixing the premix according to claim 10 with a photoinitiator, followed by photocuring.

11. A method for preparing the hydrogel, comprising:
- a step of preparing a first solution (3) comprising 2-hydroxyethyl cellulose (4) and water (5),
- a step of preparing a second solution (6) comprising a photoinitiator (7) for curing,
- a step of preparing a first mixture (8 or 15) comprising acrylic acid (9) or N-hydroxyethyl acrylamide (14), acrylonitrile (10), ethylene glycol dimethylacrylate (11) and glycerol (12),
- a step of mixing the first solution (3), the second solution (6), and the first mixture (8 or 15) to obtain a second mixture (13 or 16),
- a step of filling a mold (23) with the second mixture (13 or 16), the second mixture (13 or 16) is composed of a first layer (140) located in a first hydrophobic material (140a), a second layer (141) located in a second hydrophobic material (141a) on the first layer (140) and a frame (142) located on the second layer; a step of closing the mold (23) with a third layer (143) located in a third hydrophobic material (143a);
- a step of pressing the mold (23) after the closing step,
- a step of synthesizing a film (1 or 2) through photopolymerization, the photopolymerization is performed through the following step;
- exposing the second mixture (13 or 16) to ultraviolet light in the mold (23) subjected to pressure and covered by the third layer (143) to obtain the hydrogel film (1 or 2) comprising polyacrylonitrile (24),
- a step of removing the third layer (143) and the mold (23).

12. A pharmaceutical composition, wherein the pharmaceutical composition comprises the hydrogel according to any one of claims 1-8 and pharmaceutical active ingredients.

13. The pharmaceutical composition according to claim 12, wherein the pharmaceutical composition is at least one of a transdermal patch, a wound care patch, a skin care patch and a tissue regeneration patch.

14. Application of the hydrogel according to any one of claims 1-8 in preparation of a sensor, an actuator, a transistor, a capacitor, a garment material and a battery.
